# EUROPEAN PATENT APPLICATION

(11) **EP 2 290 395 A2**
(43) Date of publication of application: **02.03.2011**
(21) Application number: 10173223.8
(22) Date of filing: 18.08.2010
(51) Int. Cl.: G01S 7/52

(54) **Ultrasound system and method for providing information indicative of a change of elasticity information**

(30) Priority: 20.08.2009 KR 20090077177
(71) Applicant: Medison Co., Ltd., Kangwon-do 250-875 (KR)
(72) Inventor: Shin, Dong Kuk, 135-851 Seoul (KR); Yang, Eun Ho, 135-851 Seoul (KR); Hyun, Dong Gyu, 135-851 Seoul (KR)
(74) Representative: Schmid, Wolfgang

(57) **Abstract**

Embodiments for providing information indicative of a change in elasticity information of a target object over time in an ultrasound system are disclosed. In one embodiment, an ultrasound data acquisition unit transmits ultrasound beams to a target object and receives ultrasound echoes reflected from the target object to thereby provide plural sets of ultrasound frame data. An image forming unit forms ultrasound images by using the plural sets of ultrasound frame data. An information forming unit defines a region of interest on each of the ultrasound images and compares images within the regions of interest between the neighboring ultrasound images to form elasticity information. The information forming unit forms information indicative of a change in the elasticity information over time.

## Description

### CROSS-REFERENCE TO RELATED APPLICATIONS

The present application claims priority from Korean Patent Application No. 10-2009-0077177 filed on August 20, 2009, the entire subject matter of which are incorporated herein by reference.

### FIELD

Embodiments described herein generally relate to an ultrasound system, and more particularly to an ultrasound system and a method for providing information indicative of a change in elasticity information of a target object over time.

### BACKGROUND

An ultrasound system has become an important and popular diagnostic tool since it has a wide range of applications. Specifically, due to its non-invasive and non-destructive nature, the ultrasound system has been extensively used in the medical profession. Modem high-performance ultrasound systems and techniques are commonly used to produce two or three-dimensional images of internal features of an object (e.g., human organs).

Generally, the typical ultrasound system may have a function of displaying a brightness-mode (B-mode) image. The B-mode image may be formed by using reflectivity caused by an acoustic impedance difference between the tissues of the target object. However, if the reflectivity of the target object is hardly different from those of the neighboring tissues such as tumor, cancer or the like, then it is not easy to recognize the target object in the B-mode image.

To cope with the above problem, an ultrasound elastic imaging technology has been developed to display an image of the target object by using mechanical characteristics of the target object such as the elasticity of the target object. The ultrasound elastic imaging technology has been found upon the physical observations that the tumor or cancer is relatively stiffer than the neighboring tissues and, when stress is uniformly applied, a displacement of the tumor or cancer is typically smaller than those of the neighboring tissues. This technology was proven to be very helpful for diagnosing lesions such as tumor and cancer.

Conventionally, the ultrasound system may provide elastic images based on elasticity information between neighboring ultrasound image data. Thus, elasticity changes of the target object over time may not be observed through the elastic images.

### SUMMARY

Embodiments for providing information indicative of a change in elasticity information of a target object in an ultrasound system are disclosed herein. In one embodiment, by way of non-limiting example, an ultrasound system comprises: an ultrasound data acquisition unit configured to transmit ultrasound beams to a target object and receive ultrasound echoes reflected from the target object to thereby provide plural sets of ultrasound frame data; an image forming unit configured to form ultrasound images by using the plurality of ultrasound frame data; and an information forming unit configured to define a region of interest on each of the ultrasound images and compare images within the regions of interest between the neighboring ultrasound images to form elasticity information, the information forming unit being further configured to form information indicative of a change in the elasticity information over time.

In another embodiment, a method of providing indicative of a change in elasticity information of a target object over time in an ultrasound system, comprises: transmitting ultrasound beams to a target object and receiving ultrasound echoes reflected from the target object to thereby provide plural sets of ultrasound frame data; forming ultrasound images by using the plural sets of ultrasound frame data; defining a region of interest on each of the ultrasound images; comparing images within the regions of interest between the neighboring ultrasound images to form elasticity information; and forming information indicative of a change of elasticity information over time.

The Summary is provided to introduce a selection of concepts in a simplified form that are further described below in the Detailed Description. This Summary is not intended to identify key or essential features of the claimed subject matter, nor is it intended to be used in determining the scope of the claimed subject matter.

### BRIEF DESCRIPTION OF THE DRAWINGS

FIG. 1 is a block diagram showing an illustrative embodiment of an ultrasound system.
FIGS. 2 to 5 are diagrams showing illustrative embodiments of defining a region of interest in an ultrasound system.
FIG. 6 is a diagram showing an illustrative embodiment of an electrocardiogram signal.
FIGS. 7 to 8 are diagrams showing illustrative embodiments of defining a region of interest in an ultrasound system.

### DETAILED DESCRIPTION

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure.

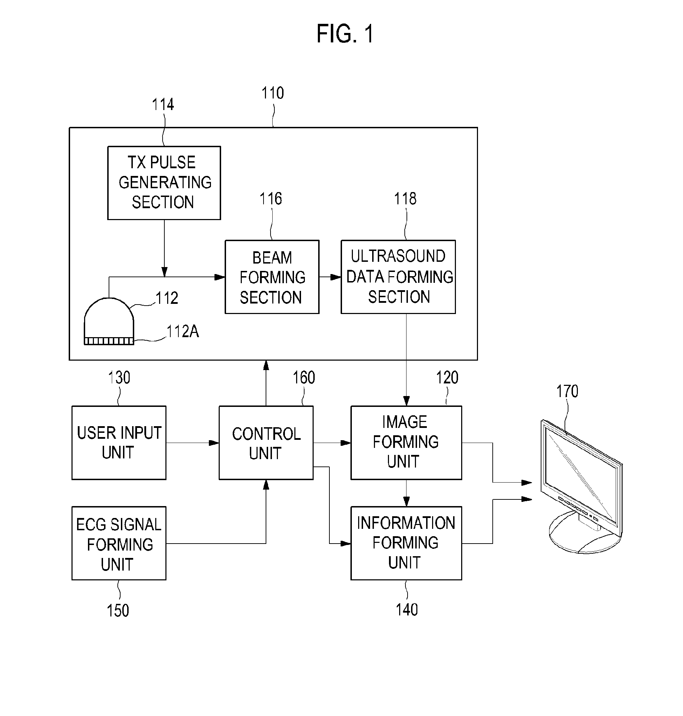

Referring to FIG. 1, an ultrasound system 100 constructed in accordance with one embodiment is shown. The ultrasound system 100 may include an ultrasound data acquisition unit 110. The ultrasound data acquisition unit 110 may be configured to transmit ultrasound beams to a target object and receive ultrasound echoes reflected from the target object to thereby form ultrasound data representative of the target object.

The ultrasound data acquisition unit 110 may include an ultrasound probe 112 containing an array transducer 112A. The array transducer 112A, which has a plurality of transducer elements, may be configured to transmit ultrasound beams to a target object. In one embodiment, the ultrasound probe 112 may include any one of a linear probe, a convex probe and the like.

The transmission of the ultrasound beams may be controlled by a transmission (Tx) pulse generating section 114, which is coupled to the ultrasound probe 112. The Tx pulse generating section 114 may include a plurality of pulsers to generate Tx pulses, which are delivered to the transducer elements of the array transducer for actuation thereof. The Tx pulse generating section 114 may be further operable to apply delays to the Tx pulses to form a Tx pattern, by which the actuation of the transducer elements may be controlled. The delays may be determined by considering distances between focal points and the respective transducer elements.

The transducer elements of the ultrasound probe 112 may receive ultrasound echoes reflected from the target object and then output electrical receive signals. The ultrasound data acquisition unit 110 may further include a beam forming section 116, which is coupled to the ultrasound probe 112. The beam forming section 116 may be operable to digitize the electrical receive signals to obtain digital signals. The beam forming section 116 may be further operable to apply delays to the digital signals in consideration of distances between the transducer elements of the ultrasound probe 112 and the focal points. The beam forming section 116 may further sum the delayed digital signals to form receive-focused beams.

The ultrasound data acquisition unit 110 may further include an ultrasound data forming section 118, which is coupled to the beam forming section 116. The ultrasound data forming section 118 may be operable to form ultrasound frame data based on the receive-focused beams. The ultrasound frame data may be radio frequency data, In-phase/Quadrature data and the like. Further, the ultrasound data acquisition unit 110 may be configured to perform a variety of signal processing upon the receive-focused beams such as gain adjustment, filtering and the like, which are necessary in forming the ultrasound frame data.

The ultrasound system 100 may further include an image forming unit 120 that may be operable to form ultrasound images based on the ultrasound frame data, which are sequentially provided from the ultrasound data acquisition unit 110. In one embodiment, the ultrasound images may include an elastic image, a brightness-mode (B-mode) image and a 3-dimensional ultrasound image. However, it should be noted that the ultrasound images may not be limited thereto.

The ultrasound system 100 may further include a user input unit 130 allowing a user to input user instructions. In one embodiment, the user instructions may include an instruction for defining a region of interest (hereinafter, referred to as "ROI instruction") in the ultrasound image. The user input unit 130 may include at least one of a control panel, a mouse, a keyboard and the like.

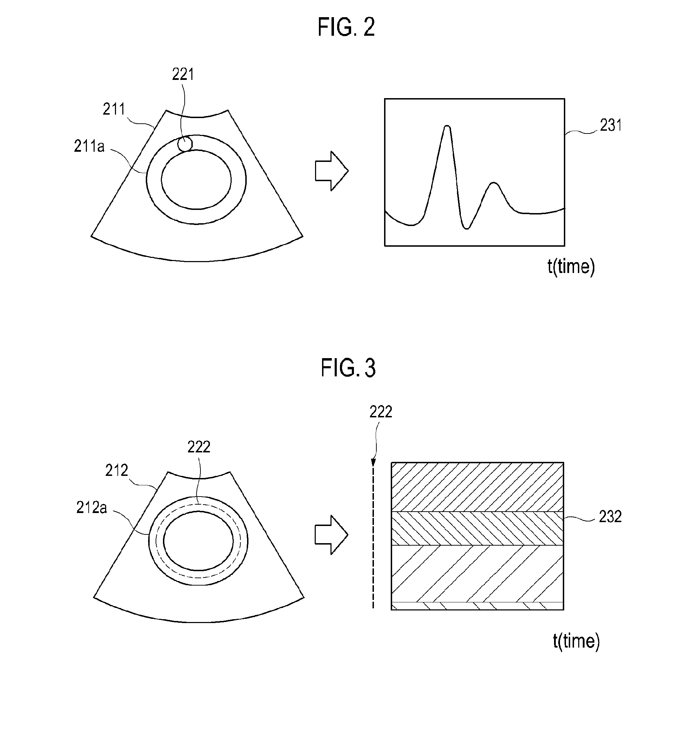

The ultrasound system 100 may further include an information forming unit 140 that may be operable to form elasticity information based on the ultrasound images. If the ROI instruction is inputted through the user input unit 130, then the information forming unit 140 may be operable to define a ROI 221 in an image of the target object, e.g., a vessel image 221 a, which constitutes the main part of each of the ultrasound images 211, as shown in FIG. 2. In such a case, the ultrasound images may be B-mode images. The information forming unit 140 may be operable to compare images within the ROIS of the neighboring ultrasound images, i.e., an N^{th} ultrasound image and an (N+1)^{th} ultrasound image to compute displacements between the images, wherein N is a positive integer equal to or greater than 1. The displacements may be computed by using correlation, e.g., cross correlation between the images. In one embodiment, prior to computing the displacements, the information forming unit 140 may be further operable to estimate a motion of the target object for the motion compensation. The motion estimation may be performed by well-known methods such as a block matching method and the like. As such, detailed descriptions thereof will be omitted herein. The information forming unit 140 may be further operable to form information on elasticity, e.g., strain or stress (elasticity information), based on the computed displacements. The information forming unit 140 may be further operable to form information indicative of a change in the elasticity information at the ROIS over time. The elasticity change information may be indicated by a graph showing a change of the elasticity information over time, as shown in FIG. 2.

In one embodiment, the ROI 221 may be defined inside of the vessel and along the contour of the vessel 221 a in each of the ultrasound images 212 in response to the ROI instruction, as shown in FIG. 3. The information forming unit 140 may be operable to compute displacements and form elasticity information based on the computed displacements in a similar manner as above. The information forming unit 140 may be further operable to form information indicative of a change in the elasticity information over time. In one embodiment, the information indicative of the change in the elasticity information may be indicated by various colors (indicated by different types of hatching in FIG. 3).

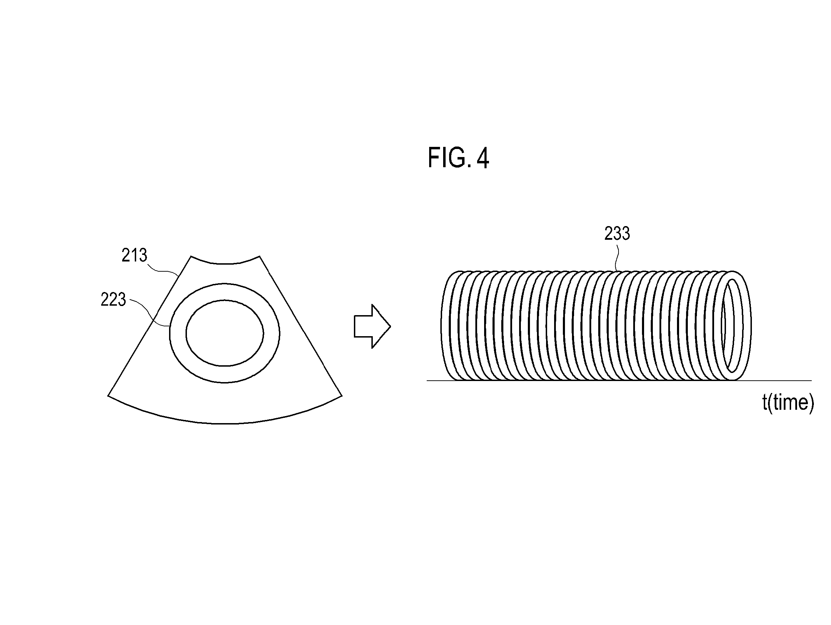

In one embodiment, the ROI 223 may be defined to be identical to the contour of the vessel image in each of ultrasound images 213 in response to the ROI instruction, as shown in FIG. 4. The information forming unit 140 may be operable to compute displacements and form elasticity information based on the computed displacements in a similar manner to the embodiment described in conjunction with FIG. 2. The information forming unit 140 may be further operable to form elasticity change information 233 in a vessel-shape, which may show a change in the elasticity of the target object such as strain or stress over time.

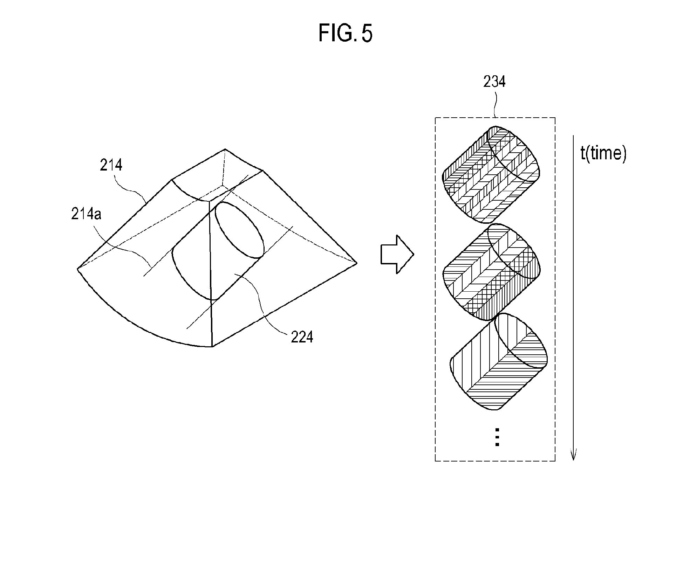

In one embodiment, the information forming unit 140 may be operable to define a ROI 224 having a 3-dimensional shape, such as a cylinder shape, within the vessel image 214a in a 3-dimensional ultrasound image 214 in response to the ROI instruction, as shown in FIG. 5. The information forming unit 140 may be operable to compute displacements and form elasticity information based on the computed displacements in a similar manner to the embodiment described in conjunction with FIG. 2. The information forming unit 140 may be further operable to form information 234 on a change in the elasticity information with various colors (indicated by different types of hatching in FIG. 5) over time.

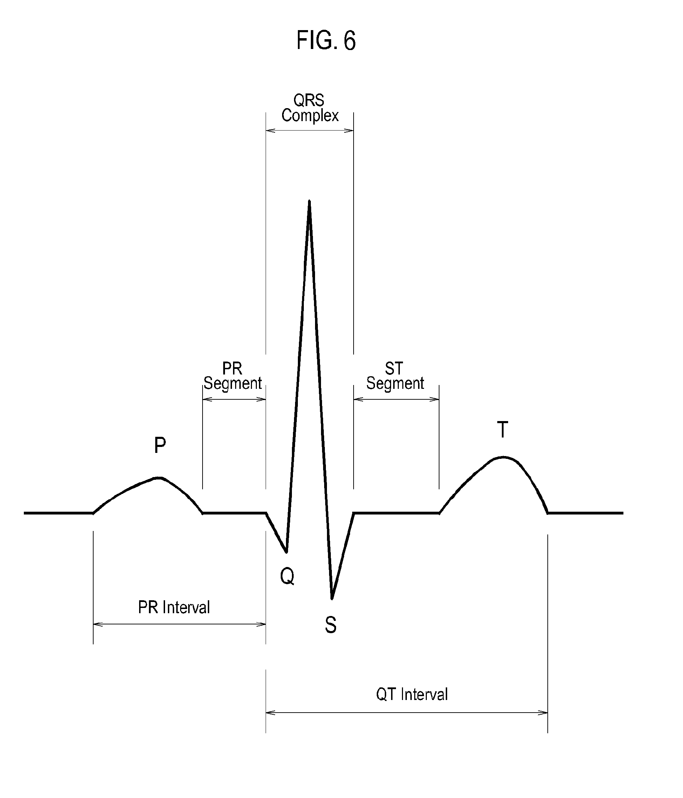

The ultrasound system 100 may further include an electrocardiogram (ECG) signal forming unit 150, which may be operable to form an ECG signal based on minute electrical changes occurring during the heartbeat, as shown in FIG. 6. The ECG signal may include a P signal, an R signal, a Q signal, an S signal and a T signal. The ultrasound data acquisition unit 110 may be operable to transmit and receive the ultrasound signals in synchronization with the ECG signal, thereby forming the ultrasound data synchronized with the ECG signal.

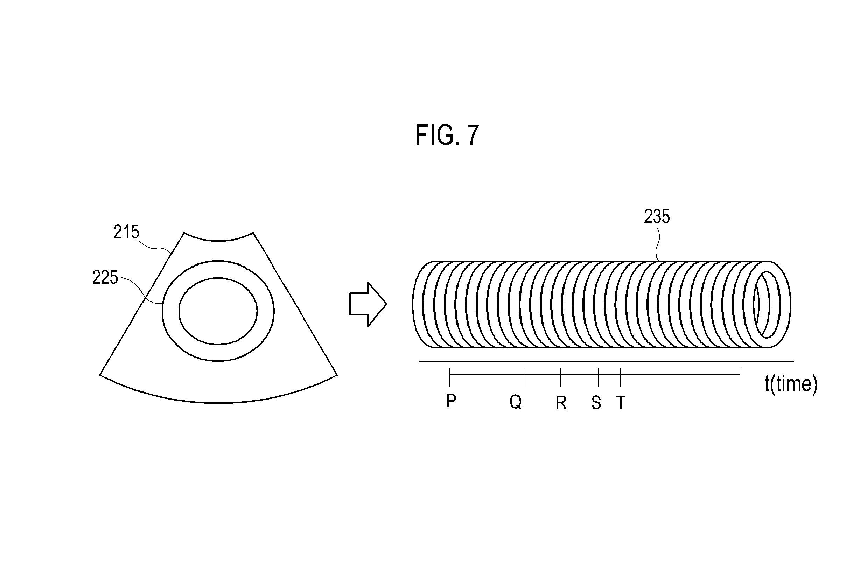

In one embodiment, the information forming unit 140 may be operable to define the ROI 225 identical to a contour of the vessel image in each of ultrasound images 215, which are formed in synchronization with the ECG signal, in response to the ROI instruction, as shown in FIG. 7. The information forming unit 140 may be operable to compare images within ROIS of the neighboring ultrasound images, i.e., an N^{th} ultrasound image and an (N+1)^{th} ultrasound image to compute displacements, wherein N is a positive integer equal to or greater than 1. The N^{th} ultrasound image and (N+1)^{th} ultrasound image may be ultrasound images, which are formed at a same type of signal, such as a P signal, an R signal, a Q signal, an S signal or a T signal, in the ECG signal. Prior to computing the displacements, the information forming unit 140 may be further operable to estimate a motion of the target object for motion compensation. The information forming unit 140 may be operable to form elasticity information based on the computed displacements. The information forming unit 140 may be further operable to form information indicative of a change in elasticity information 235 of a vessel-shape with being synchronized with the ECG signal over time.

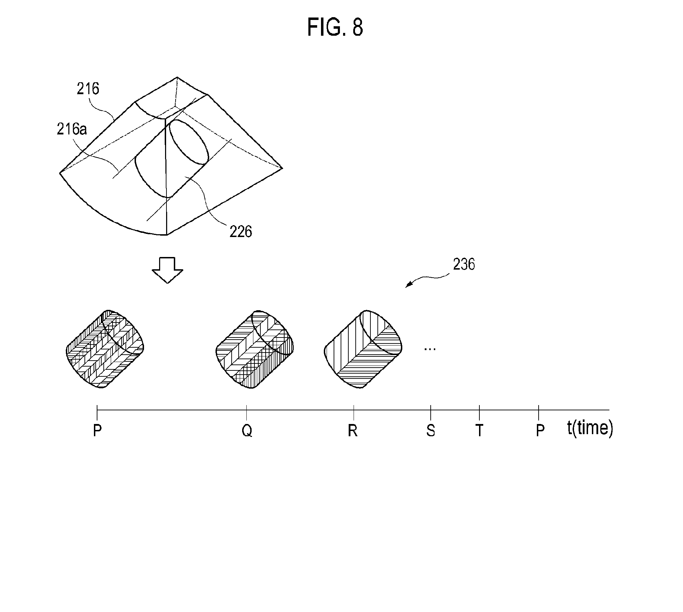

In one embodiment, the information forming unit 140 may be operable to define a ROI 226 having a 3-dimensional shape, such as a cylinder shape, within a vessel image 216a in a 3-dimensional ultrasound image 216 in response to the ROI instruction, as shown in FIG. 8. The information forming unit 140 may be operable to compute displacements and form elasticity information based on the computed displacements in a similar manner to the embodiment described in conjunction with FIG. 7. The information forming unit 140 may be further operable to form information indicative of a change in elasticity information 236 with various colors (indicated by different types of hatching in FIG. 8) with being synchronized with the ECG signals over time.

Although it has been described that the information forming unit 140 forms the information indicative of the change of the elasticity information at the same type of signal, the formation of the information may not be limited thereto. In one embodiment, the information forming unit 140 may be operable to form the information indicative of the change in the elasticity information, which may include at least one of a mean value, an intermediate value and a variation of elasticity information, at a specific interval in the ECG signal. The specific interval may include a PQ signal interval, a QR signal interval, an RS signal interval, an ST signal interval, a PR interval, a PR segment interval, a QRS complex interval, an ST segment interval and an QT interval.

The ultrasound system 100 may further include a control unit 160, which may be configured to control the operations of elements of the ultrasound system 100 such as the ultrasound data acquisition unit 110, the image forming unit 120, the information forming unit 140 and the like. The control unit 160 may be coupled to the user input unit 130 to receive the user instructions. The control unit 160 may be operable to enable the elements of the ultrasound system 100 to operate appropriately in response to user instructions.

The ultrasound system may further include a display unit 170 for displaying the ultrasound images, the elasticity information and the like. In one embodiment, the display unit 170 may include at least one of a cathode ray tube (CRT) display, a liquid crystal display (LCD), an organic light emitting diode (OLED) display and the like.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system, comprising:
an ultrasound data acquisition unit configured to transmit ultrasound beams to a target object and receive ultrasound echoes reflected from the target object to provide plural sets of ultrasound frame data;
an image forming unit coupled to the ultrasound data acquisition unit and
being configured to form ultrasound images by using the plural sets of ultrasound frame data; and
an information forming unit coupled to the image forming unit and being configured to define a region of interest on each of the ultrasound images and
compare images within the regions of interest between the neighboring ultrasound images to form elasticity information, the information forming unit being further configured to form information indicative of a change in the elasticity information over time.

2. The ultrasound system of Claim 1, further comprising a user input unit allowing a user to input user instructions to define the region of interest in the ultrasound images.

3. The ultrasound system of Claim 1, wherein the information forming unit is configured to estimate a motion of the target object in the ultrasound images for motion compensation.

4. The ultrasound system of Claim 1, further comprising an electrocardiogram signal forming unit configured to form an electrocardiogram (ECG) signal having multiple types of signals from the target object.

5. The ultrasound system of Claim 4, wherein the ultrasound data acquisition unit is configured to transmit and receive the ultrasound signals in synchronization with the ECG signal to acquire ultrasound frame data synchronized with the ECG signal, and wherein the image forming unit is configured to form the ultrasound images synchronized with the ECG signal.

6. The ultrasound system of Claim 5, wherein the information forming unit is configured to compare ultrasound images within the ROIS of the neighboring ultrasound images obtained at a same type of signal in the ECG signal to form the elasticity information and form information indicative of a change in the elasticity information in synchronization with the ECG signal over time.

7. The ultrasound system of Claim 5, wherein the information forming unit is configured to form the information including at least one of a mean value, an intermediate value and a variation of the elasticity information at a predetermined interval in the ECG signal.

8. A method of providing information indicative of a change in elasticity information of a target object over time in an ultrasound system, comprising:
a) transmitting ultrasound beams to a target object and receiving ultrasound echoes reflected from the target object to provide plural sets of ultrasound frame data;
b) forming ultrasound images by using the plural sets of ultrasound frame data;
c) defining a region of interest on each of the ultrasound images;
d) comparing images within the regions of interest between the neighboring ultrasound images to form elasticity information; and
e) forming information indicative of a change in the elasticity information over time.

9. The method of Claim 8, further comprising inputting user instructions to define the region of interest in the ultrasound images.

10. The method of Claim 8, wherein the step d) includes estimating motion of the target object in the ultrasound images for motion compensation.

11. The method of Claim 8, further comprising forming an electrocardiogram (ECG) signal having multiple types of signals from the target object.

12. The method of Claim 11, wherein the step a) includes:
transmitting and receiving the ultrasound signals in synchronization with the ECG signal to acquire ultrasound frame data synchronized with the ECG signal; and
forming the ultrasound images synchronized with the ECG signal by using the synchronized ultrasound frame data.

13. The method of Claim 12, further comprising:
comparing ultrasound images within the ROIS of the neighboring ultrasound images obtained at a same type of signal in the ECG signal to form the elasticity information; and
forming the information indicative of the change in the elasticity information in synchronization with the ECG signal.

14. The method of Claim 12, further comprising forming the elasticity information including at least one of a mean value, an intermediate value and a variation of the elasticity information at a predetermined interval in the ECG signal.

15. The method of Claim 8, further comprising displaying the information indicative of the change of the elasticity information.
